# EUROPEAN PATENT SPECIFICATION

(11) **EP 4 448 081 B1**
(45) Date of publication and mention of the grant of the patent: **08.10.2025**
(21) Application number: 22859529.4
(22) Date of filing: 19.12.2022
(51) Int. Cl.: A61N 1/06, A61N 1/05, A61N 1/36

(54) **ELECTROSTIMULATION DEVICE WITH A DOUBLE PERCUTANEOUS COAXIAL NEEDLE AND PROCESS FOR MANUFACTURING THE SAME**
ELEKTROSTIMULATIONSVORRICHTUNG MIT DOPPELTER PERKUTANER KOAXIALNADEL UND HERSTELLUNGSVERFAHREN DAFÜR
DISPOSITIF D'ÉLECTROSTIMULATION À DOUBLE AIGUILLE COAXIALE PERCUTANÉE ET SON PROCÉDÉ DE FABRICATION

(30) Priority: 17.12.2021 IT 202100031724
(43) Date of publication of application: 23.10.2024
(73) Proprietor: Forino, Luciano, 10044 Pianezza (TO) (IT)
(72) Inventor: Forino, Luciano, 10044 Pianezza (TO) (IT)
(74) Representative: Aprà, Mario
(86) International application number: PCT/IB2022/062488
(87) International publication number: WO 2023/112006

(56) References cited:
- WO-A1-2014/074237
- US-A1- 2017 049 507
- US-B1- 7 201 731

## Description

The present invention refers to an electrostimulation device with a double percutaneous coaxial needle, in particular for minimally invasive applications of percutaneous radiofrequency in the treatment of chronic pain in hospital settings. Said electrostimulation device with double percutaneous coaxial needle will be referred to hereinafter as "device" for the sake of brevity. The invention also refers to the process for manufacturing the device specified above.

The Italian patent N. 102016000089091 of the same inventor describes a device of the specified type, in which a transcutaneous monopolar needle means that can be stimulated and thermodamaging is electrically connected in an electric circuit with respect to an electric pulse generator and to a neutral dispersion plate, so-called ground electrode, designed to close the electric circuit between a negative pole and a positive pole, for minimally invasive applications of percutaneous radiofrequency in the treatment of chronic benign pain in hospital settings.

Said needle means comprises two coaxial tubular parts, called cannula and counter-cannula respectively, wherein the cannula is external to the counter-cannula. Cannula and counter-cannula are made of electrically conductive material, for example steel, and are coupled to each other with possibility of relative axial sliding, electrically insulated and supported, at one of their ends, the so-called proximal end, by respective axially perforated and coaxial conical support bodies (in the hereinafter "support cones"), which are made of electrically insulating material, for example plastic material. The support cones are connected to each other with possibility of relative axial sliding and, in particular, the support cone of the cannula has an internal axial cavity with a conical surface and the support cone of the counter-cannula has a conical external axial surface, configured to achieve a geometric coupling with respect to the conical cavity of the cannula support cone.

Cannula and counter-cannula are provided at their other axial end, the so-called distal end, with respective tips. The cannula has a cutting tip, suitable for skin puncture only, while the counter-cannula has a non-cutting tip.

Furthermore, the cannula is completely isolated externally by an electrically and thermally insulating coating.

The counter-cannula includes a so-called U.T.A. ("Uncoring Tip Advance") type electrode probe.

The support cone of the counter-cannula comprises electrical connector means, configured for the electrical connection of the electrode probe with respect to the electrical pulse generator. An insulated electrical conductor is electrically connected with respect to said electrical connector means and extends outside the support cone of the counter-cannula, through the conical cavity of the support cone of the cannula.

Furthermore, the support cone of the cannula and the support cone of the counter-cannula comprise respective guide and reciprocal retention means, which are configured to allow successive steps of manual advancement and retraction of the counter-cannula with respect to the cannula, by means of relative axial sliding of the support cone of the counter-cannula in relation to the support cone of the cannula, maintaining the counter-cannula in a corresponding stable relative arrangement with respect to the cannula at each step. Specifically, in a first relative arrangement of the counter-cannula with respect to the cannula, the cutting tip of the cannula can perform skin penetration into a patient's body. In a second and subsequent relative arrangement of the counter-cannula with respect to the cannula, the tip of the counter-cannula protrudes at least partially with respect to the tip of the cannula and overlaps the cutting edge of the cannula itself, which is thus made no longer sharp, while the tip of the counter-cannula is arranged for the treatment. In at least a third and further relative arrangement of the counter-cannula with respect to the cannula, the counter-cannula is further advanced with respect to the tip of the cannula, and the needle means, including the cannula and the counter-cannula, is advanced into the subcutaneous planes and structures of the patient, until the operating target, without generating any type of accidental complication.

The device according to the aforementioned patent has the following drawbacks:
- the guide and reciprocal retention means provided in the support of the cannula and in the support of the counter-cannula are also limited in their axial stroke by the presence of the insulated electric conductor. The support body of the cannula cannot be made to the desired length. In fact, an axial lengthening of the support body of the cannula, while it allows to increase the number of stable relative positions of the counter-cannula with respect to the cannula, also increases the risk of undesired erroneous positioning of the electrical conductor in the cavity of the support cone of the cannula with consequent possible device malfunctions. In fact, once the skin puncture has been performed, the U.T.A. electrode probe is advanced within the electrically isolated cannula. The relative bidirectional movement of the two support cones and, correspondingly, of the counter-cannula with respect to the cannula, allows the operator to achieve the desired operative arrangements of the cannula (in cutting or non-cutting condition) and, during the treatment phase, to choose which active tip to use, cannula tip or counter-cannula tip. Any jamming of the insulated electrical conductor, which is moved together with the support cone of the counter-cannula into the internal cavity of the support cone of the cannula, can hinder correct operation;
- on the other hand, the insulated electric conductor occupies part of the cavity of the cannula support body, partly passes through this cavity and emerges from it in a substantially axial direction. This arrangement of the insulated electrical conductor complicates both the structure and the manufacturing process of the percutaneous coaxial needle device.

Document WO 2014/074237 A1 discloses an electrostimulation device with a double percutaneous coaxial needle having the characteristics of the pre-characterizing part of claim 1.

The present invention intends to remedy the aforementioned drawbacks of the prior art and provide a device of the type specified which allows to improve the use of the device itself and its production.

An object of the present invention is to provide an electrostimulation device with a double percutaneous coaxial needle, in particular for minimally invasive percutaneous radiofrequency applications in the treatment of chronic pain in hospitals, which allows the above applications to be performed correctly and effectively.

Another object of the present invention is to provide a device of the type specified which is improved, both functionally and structurally.

Another object of the present invention is also to provide a process for manufacturing an electrostimulation device with a double percutaneous coaxial needle of the type specified, which is easy and reliable to implement.

In view of these objects, the present invention provides an electrostimulation device with a double percutaneous coaxial needle, the essential characteristics of which form the subject-matter of claim 1.

The invention also provides a process for the manufacturing an electrostimulation device with double percutaneous coaxial needle, the essential characteristics of which form the subject of claim 8.

Other advantageous features of the present invention are described in the dependent claims.

Further features and advantages of the invention will be apparent from the following detailed description of an example of embodiment, with reference to the drawing which shows important details for the invention, and from the claims.

The features illustrated here must not necessarily be considered to scale and are represented in such a manner that the particular characteristics according to the invention are clearly highlighted.

The various features can be produced individually or in any combination with one another, as variants of the invention.

In the drawing:

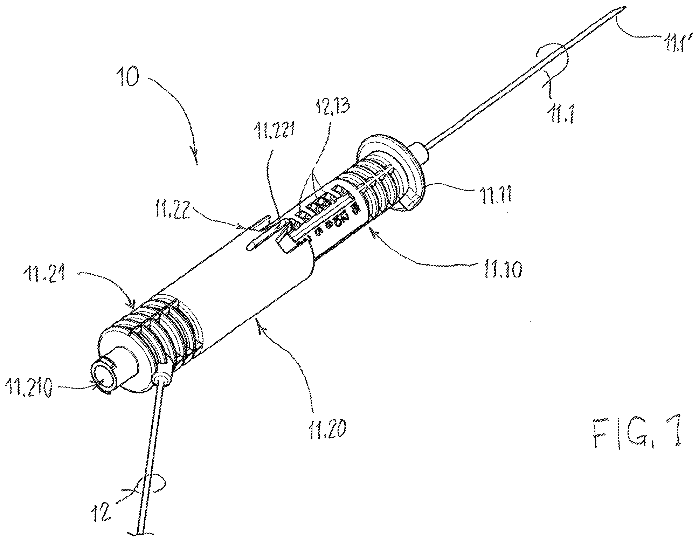

- figure 1 is a perspective view of the electrostimulation device with double percutaneous coaxial needle, in a monopolar version, according to a first exemplifying embodiment of the invention, comprising a cannula needle with relative support and a counter-cannula needle with relative support illustrated mutually connected, in which the cannula needle with relative support is interconnected and partially extended with respect to the counter-cannula needle with relative support and the device is seen from the rear;

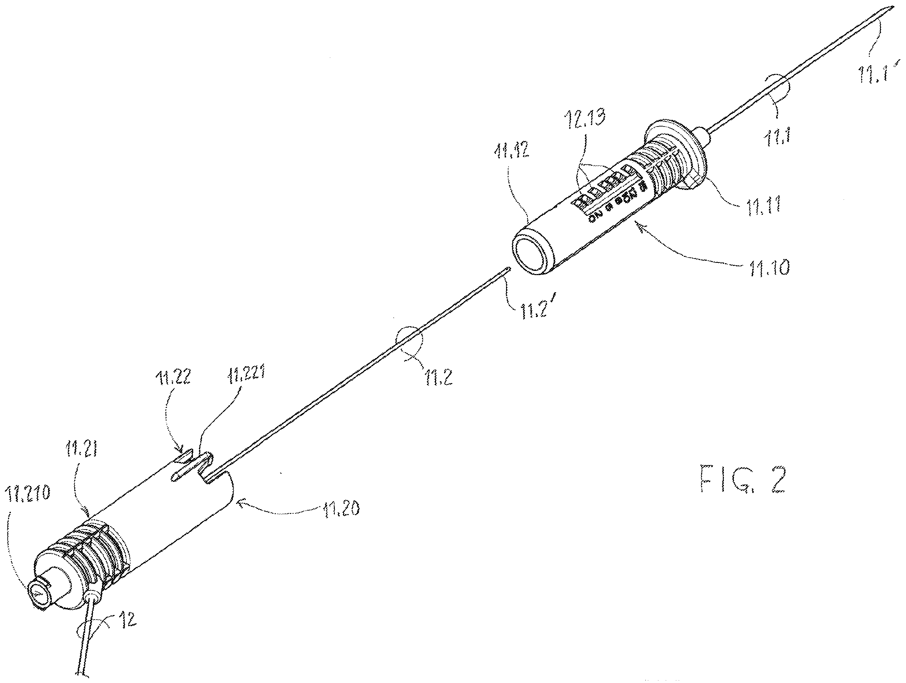

- figure 2 is a view similar to that of figure 1, but in which the device is illustrated in exploded view, in which the cannula needle with its support is separate from the counter-cannula needle with its support;

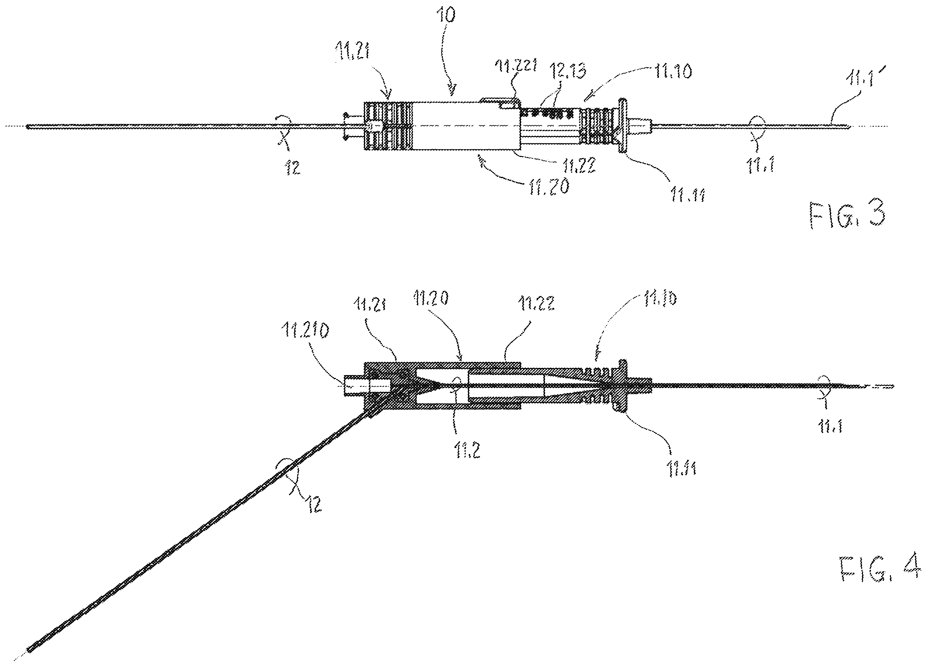

- figure 3 is an elevation view of the device of figure 1, on a different scale;
- figure 4 is an axial sectional view of the device of figure 3;

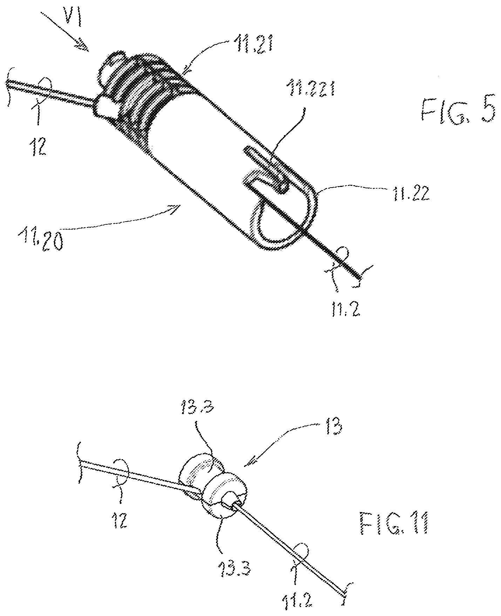

- figure 5 is a perspective view, from the front, of the counter-cannula needle of figure 2 (interrupted for clarity of illustration) with relative support, in different scales;

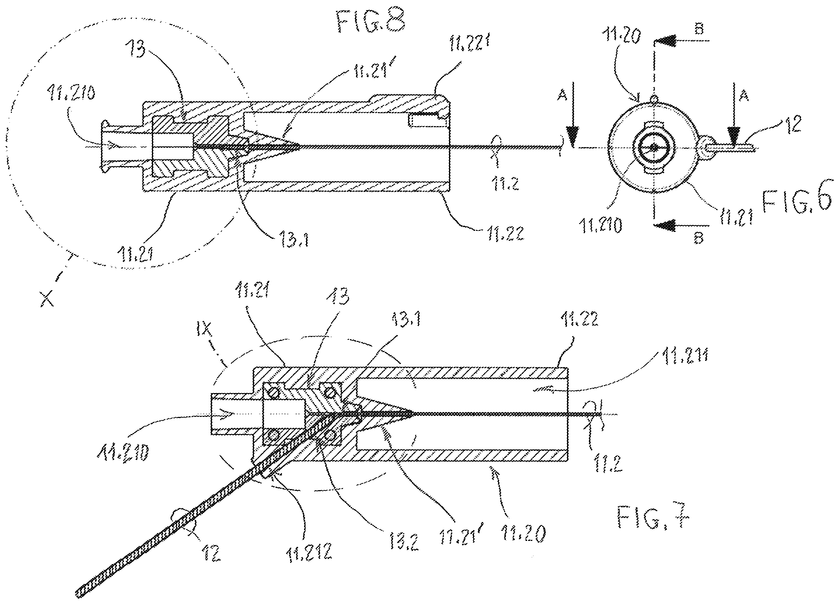

- figure 6 is a view of the support of the counter-cannula needle in the direction of arrow VI of figure 5, on a different scale;
- figure 7 is a sectional view along the line A-A of figure 6;
- figure 8 is a sectional view along the line B-B of figure 6;

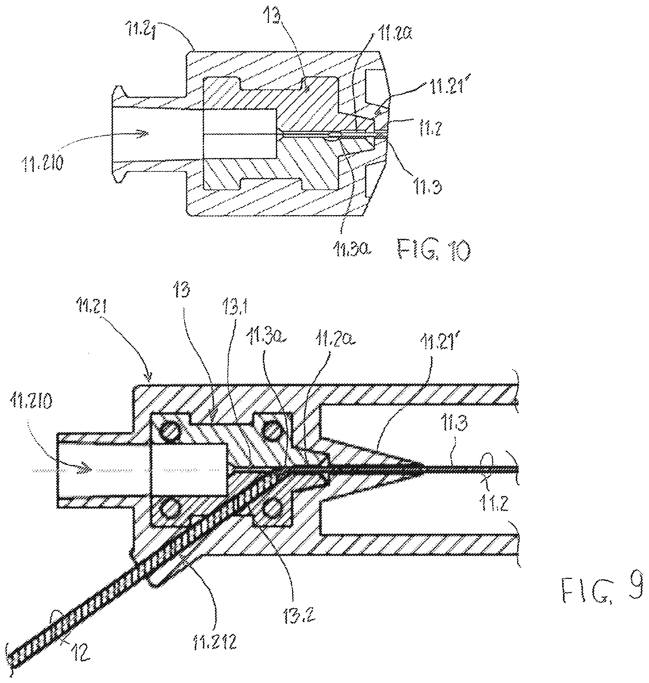

- figure 9 is a detail view, on a larger scale, of detail IX of figure 7;
- figure 10 is a detail view, on a larger scale, of detail X of figure 8;
- figure 11 is a perspective view, on a different scale, of a mounting body of the counter-cannula needle and of an electric conductor, said mounting body being included in the support of the counter-cannula needle, as illustrated for example in figure 9;

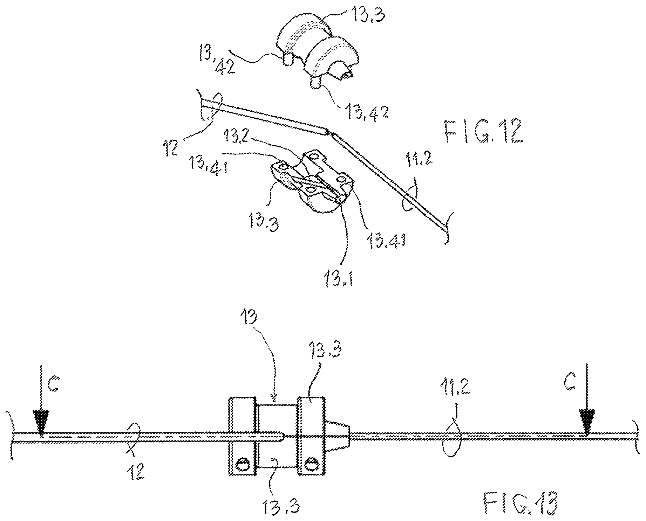

- figure 12 is a view similar to that of figure 11, but in which said mounting body is shown in an exploded view, with relative cannula needle and electric conductor;
- figure 13 is a side elevational view of the mounting body of the counter-cannula needle, with related cannula needle and electrical conductor, according to figure 11;

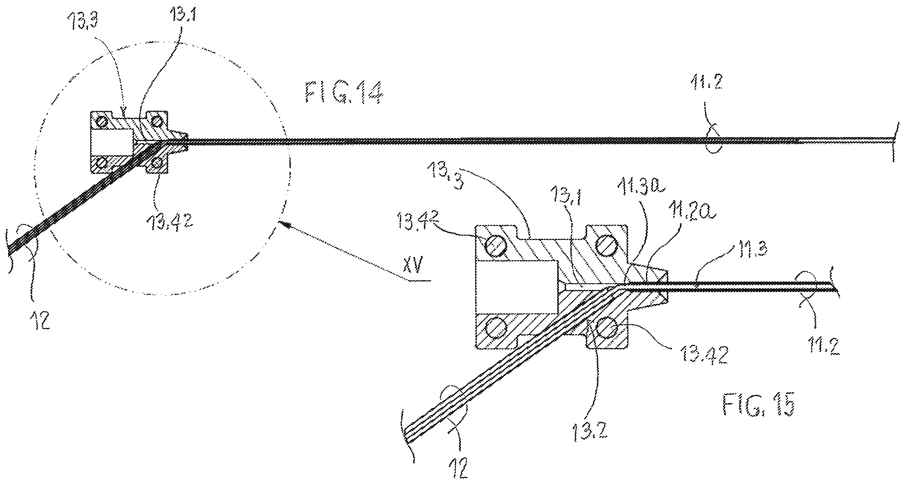

- figure 14 is a sectional view along the line C-C of figure 13, on a different scale;
- figure 15 is a detail view, on a larger scale, of detail XV of figure 14;

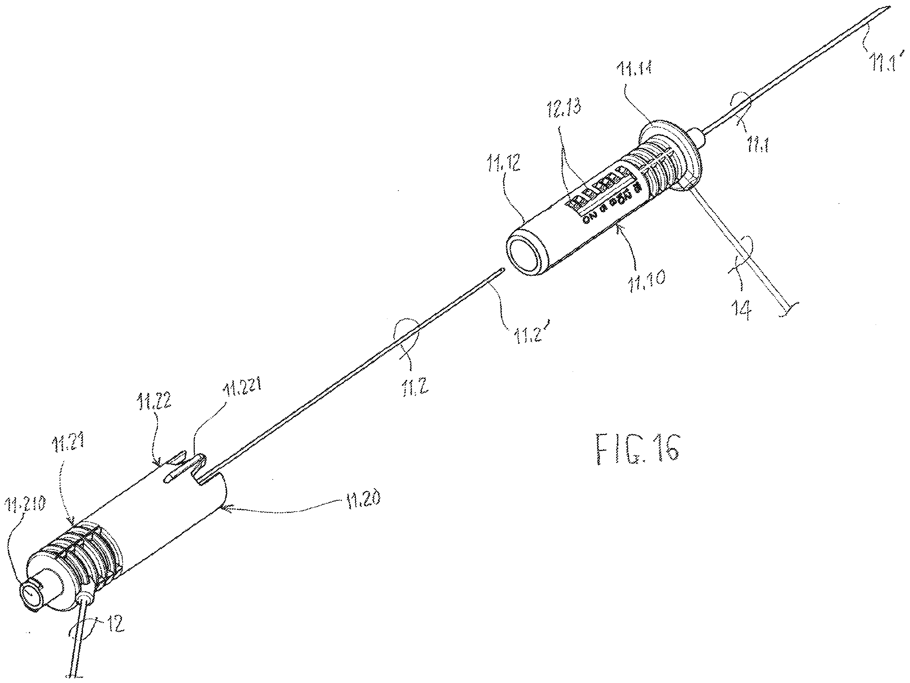

- figure 16 is a perspective view of the electrostimulation device with double percutaneous coaxial needle, in the bipolar version, according to a second exemplary embodiment of the invention.

With reference to the drawing, 10 denotes the electrostimulation device with a double percutaneous coaxial needle, according to an embodiment of the present invention. Said device 10 comprises:
- two tubular bodies 11.10, 11.20, in plastic material, hereinafter referred to respectively as cannula support 11.10 and counter-cannula support 11.20, wherein the cannula support 11.10 and the counter-cannula support 11.20 are telescopically connected with the possibility of relative axial sliding, the cannula support 11.10 being partially included within the counter-cannula support 11.20;
- a cannula needle 11.1 and a counter-cannula needle 11.2, hereinafter referred to respectively as cannula 11.1 and counter-cannula 11.2, axially perforated, in electrical conductive material, wherein the cannula 11.1 is supported fixed and coaxial with respect to the cannula support 11.10 and the counter-cannula 11.2 is supported fixed and coaxial with respect to the counter-cannula support 11.20 and wherein the counter-cannula 11.2 is inserted in a coaxial arrangement and is electrically insulated with respect to the cannula 11.1, with the possibility of relative axial sliding.

The cannula support 11.10 is configured, at a first axial end zone 11.11, so-called front end, as a bottom having an axial through hole, with respect to which an axial end of said cannula is fixed 11.1, the remainder of the cannula 11.1 extending beyond said front end 11.11 and towards the outside of the cannula support 11.10.

The cannula 11.1 has a sharp tip 11.1', distal with respect to said front end 11.11 of the cannula support 11.10 and suitable for skin puncture.

The counter-cannula support 11.20 comprises an integral internal diaphragm 11.21' provided in proximity to a first axial end zone 11.21, so-called rear end, distal with respect to said front end 11.11 of the cannula support 11.10.

Said integral internal diaphragm 11.21' comprises a through axial hole and defines in said counter-cannula support 11.20:
- a first axial cavity 11.210, so-called rear cavity, open towards the outside at said rear end 11.21, wherein said rear cavity 11.210 has one internal side wall contiguous to said diaphragm 11.21',
- a second axial cavity 11.211, so-called front cavity, open towards the outside at a second axial end zone 11.22 of said counter-cannula support 11.20, axially opposite to said rear end 11.21, wherein said cannula support 11.10 is inserted into said front cavity 11.211, by means of a second axial end zone 11.12 of said cannula support 11.10, axially opposite to said front end 11.11, with the possibility of relative axial sliding together with the respective cannula 11.1.

It is highlighted that the counter-cannula 11.2:
- is fixed, in a coaxial arrangement, and with an intermediate portion, with respect to the axial hole of said internal diaphragm 11.21' of the counter-cannula support 11.20;
- extends into and beyond said front cavity 11.211 of said counter-cannula support 11.20;
- has a non-sharp tip 11.2', at a distal end with respect to said diaphragm 11.21' of the counter-cannula support 11.20, arranged beyond said counter-cannula support 11.20 and configured to extend outwards and inwards with respect to the sharp tip 11.1' of the cannula 11.1;
- has an axial end 11.2a, proximal to said diaphragm 11.21' opposite said non-sharp tip 11.2', extending beyond said diaphragm 11.21' in said rear cavity 11.210.

Furthermore, said device 10 comprises a mounting body 13 of said counter-cannula 11.2 with respect to said counter-cannula support 11.20, which is fixed in said rear cavity 11.210 of the counter-cannula support 11.20 and has at least one outer side wall facing towards and contiguous with respect to said inner side wall of said rear cavity 11.210 of said counter-cannula support 11.20.

It is highlighted that said mounting body 13 is made of electrically insulating material, for example in plastic material, includes a first hole 13.1, which is axially aligned with said axial hole of the diaphragm 11.21' of the counter-cannula support 11.20, and comprises a second through hole 13.2, lateral with respect to said first hole 13.1 and extending between said first hole 13.1, on which the hole 13.2 is open, and said at least one outer side wall of said mounting body 13, on which the hole 13.2 itself is open.

The counter-cannula 11.2 has said axial end 11.2a sealingly inserted in said first hole 13.1 of said mounting body 13 and comprises a thermocouple means 11.3, fixed and coaxially arranged therein. Said thermocouple means 11.3 has a first electrically conductive axial end 11.3a, arranged outside the axial end 11.2a of the counter-cannula 11.2 and inside said first hole 13.1 of the mounting body 13.

A first flexible insulated electric conductor 12 has an intermediate portion inserted in said second through hole 13.2 of the mounting body 13 and a first electrically conductive end electrically connected with respect to a said first end 11.3a of said thermocouple means 11.3, while a second end of said thermocouple means 11.3, axially opposed to said first end 11.3a, is arranged at said non-sharp tip 11.2' of the counter-cannula 11.2 and is fixed to said non-sharp tip 11.2' of said counter-cannula 11.2.

Said counter-cannula support 11.20 comprises, in said rear end 11.21, a through hole 11.212, whose axis is axially aligned with the axis of said second hole 13.2 of said mounting body 13. Said first insulated electrical conductor 12 has a further portion inserted in said through hole 11.212 of the counter-cannula support 11.20 and is branched outside the counter-cannula support 11.20. Said first insulated electrical conductor 12 is electrically connected, by means of a second electrically conductive end, with respect to an external electrical control circuit (which is known and not explained). By means of the aforesaid arrangement, said counter-cannula 11.2 is configured as U.T.A. ("Uncoring Tip Advance") Electrode Probe, whose primary function is to adjust the active tip of the device 10, suitable for the procedure, and also to make the cannula 11.1 of the non-coring type. The U.T.A. Electrode Probe itself comprises said thermocouple means 11.3 suitable for monitoring and detecting the electrical functions of the electrical control circuit and for the correct operation of the procedure, in relation to the basic parameters set by a doctor using an electrical pulse generator device (per se known and not illustrated), so-called "auxiliary RF generator", included in said external electric control circuit.

For example, in a monopolar configuration of the device 10, as illustrated hereabove, said external electric control circuit may comprise said electric pulse generator and a neutral dispersion plate, adapted to close the electric circuit between a negative pole and a positive pole. It is enhanced that, in this arrangement, the cannula 11.1 is electrically insulated over its entire outer surface, for example by means of an electrically insulating coating.

On the other hand, according to another embodiment illustrated in figure 18, in a bipolar configuration of the device 10, said insulated electric conductor 12 is electrically connected in a suitable external electric circuit, with respect to which also said cannula 11.1 is electrically connected by means of a first conductive end of a second flexible insulated electric conductor 14, which is electrically connected, by means of a second conductive end, at the end of said cannula 11.1 fixed with respect to said bottom in the first end zone 11.11 of the cannula support 11.10. It should be noted that, in this arrangement, the cannula 11.1 has at least one electrically conductive end portion, while it is electrically insulated, for example by means of an electrically insulating coating, over the entire remaining part of its outer surface.

By means of the aforementioned arrangement, cannula 11.1 and counter-cannula 11.2, coupled each other with the possibility of relative axial sliding, provide a transcutaneous needle means with an indifferent tip and variable in length, which can be stimulated and thermodamaging through an active tip 11.1', 11.2' disposed in the distal part with respect to said first end zone 11.11 of the cannula support 11.10.

It is enhanced that said mounting body 13 comprises two half-bodies 13.3, juxtaposed and mating at respective opposite faces in a plane containing the axes of said first hole 13.1 and said second hole 13.2 of the mounting body 13.

In particular, each of said opposite faces of the half-bodies 13.3 has a respective first channel and a respective second channel, wherein said first channel is extended over the entire length of the assembled body 13 and said second channel extends laterally and communicates with respect to said first channel. By means of this arrangement said two juxtaposed and mutually fixed half-bodies 13.3 provide, by means of said respective first channel and second channel, reciprocally superimposed, said first hole 13.1 and said second hole 13.2 of the mounting body 13.

Furthermore, said opposite faces of said two half-bodies 13.3 have respective recesses 13.41 and mounting pins 13.42, mutually complementary and opposite, which form the assembly of the mounting body 13 by means of positive and/or force coupling. By means of this arrangement, the two half-bodies 13.3 can be assembled for example by hand, to form the mounting body 13, in which said end 11.2a of the counter-cannula 11.2 is inserted in the first hole 13.1 and said portion of the first insulated electric conductor 12 in the second hole 13.2.

On the other hand, it is highlighted that said cannula support 11.10 has, on the outer side surface and near said second end zone 11.12, a linear arrangement of recesses 12.13, extending in the axial direction and configured as notches of a graduated scale. Said counter-cannula support 11.20 comprises an integral elastic finger 11.221, projecting with respect to said second end zone 11.22 of the counter-cannula support 11.20. Said finger 11.221 has an elastically flexible tip, configured for cooperating in an elastic snap with each recess of said arrangement of recesses 12.13 provided in said side surface of said cannula support 11.10.

This arrangement makes it possible to set up in the device 10 a plurality of positions of stable telescopic extension or retraction of the cannula support 11.10 with respect to the counter-cannula support 11.20, in which the cannula 11.1 and the counter-cannula 11.2 assume different predetermined relative and operative positions. To each relative position of the counter-cannula 11.2 with respect to the cannula 11.1 corresponds a respective recess of said arrangement of recesses 12.13 of the cannula support 10, in which said elastic finger 11.221 of the counter-cannula support 11.20 can be engaged (and from which it can be disengaged), as a result of manual action by an operator.

It is to be noted that the rear end of the counter-cannula support 11.20 is configured for sealing connection with liquid infusion devices, which are delivered through the counter-cannula 11.2. In particular, it should be noted that the counter-cannula support 11.20 comprises, in correspondence with said first axial end zone 11.21, a "luer lock" type connection communicating with said rear cavity 11.210 for the intrinsic infusion of liquids through the counter-cannula 11.2.

The present invention also provides a process for manufacturing an electrostimulation device 10 with double coaxial percutaneous needle, wherein said device 10 comprises:
- two tubular bodies 11.10, 11.20, in plastic material, hereinafter referred to respectively as cannula support 11.10 and counter-cannula support 11.20, wherein the cannula support 11.10 and the counter-cannula support 11.20 are telescopically connected with the possibility of relative axial sliding, the cannula support 11.10 being partially inserted in said counter-cannula support 11.20;
- a cannula needle 11.1 and a counter-cannula needle 11.2, hereinafter referred to respectively as cannula 11.1 and counter-cannula 11.2, axially perforated, in electrical conductive material, wherein the cannula 11.1 is supported fixed and coaxial with respect to the cannula support 11.10 and the counter-cannula 11.2 is supported fixed and coaxial with respect to the counter-cannula support 11.20 and wherein the counter-cannula 11.2 is inserted in a coaxial arrangement and is electrically insulated with respect to the cannula 11.1, with the possibility of relative axial sliding.

According to the present invention the aforementioned process is characterized in that it comprises the following production steps of said counter-cannula support 11.20 with relative counter-cannula 11.2 fixed with respect to said counter-cannula support 11.20:
- providing a counter-cannula 11.2, which has a first axial end 11.2a opposite a second axial end, so-called non-sharp tip 11.2';
- providing a thermocouple means 11.3, configured to be inserted and fixed in said counter-cannula 11.2 in an axial arrangement and having a first axial end 11.31, electrically conductive, and a second axial end opposite to said first end 11.31;
- inserting and fixing said thermocouple means 11.3 in an axial arrangement in said counter-cannula 11.2, placing said first axial end 11.31 of said thermocouple means 11.3 in proximity to said first axial end 11.2a of the counter-cannula 11.2 and said second axial end of said thermocouple means 11.3 in proximity to said non-sharp tip;
- providing a first insulated electrical conductor 12, hereinafter referred to as the first conductor 12, which has a first electrically conductive end and a second electrically conductive end;
- making a mounting body 13 of said counter-cannula 11.2 with respect to said counter-cannula support 11.20, wherein said mounting body 13 is made of electrically insulating material and is provided with:
   - at least one outer side wall;
   - a first hole 13.1, configured to house a first part, the so-called end part, of said axial end 11.2a of the counter-cannula 11.2,
   - a second hole 13.2, passing through and extending between said first hole 13.1 and said at least one outer side wall of said mounting body 13, wherein said second hole 13.2 is configured to house a first section of a first part of said first conductor 12, wherein said first section is contiguous to said first electrically conductive end of said first conductor 12, which is arranged outside said second hole 13.2;
- inserting said end part of said axial end 11.2a of the counter-cannula 11.2 in said first hole 13.1 of said mounting body 13, inserting said first section of said first part of said first conductor 12 in said second hole 13.2 of said mounting body 13 and juxtaposing said first electrically conductive end of said first conductor 12 and said first axial end 11.31 of said thermocouple means 11.3, making a fixed electrical contact;
- providing a mould configured for injection moulding of molten plastic material and metal and providing in said mould moulding means for said counter-cannula support 11.20, wherein said moulding means are configured as injection cavities of molten plastic material;
- arranging in said injection cavity of molten plastic material said mounting body 13 including said end part of said axial end 11.2a of said counter-cannula 11.2, said first section of said first part of said first conductor 12 and said fixed electrical contact between said first electrically conductive end of said first conductor 12 and said first axial end 11.31 of said thermocouple means 13;
- configuring said injection cavity of molten plastic material to form said counter-cannula support 11.20 as a tubular body and to form in said counter-cannula support 11.20:
   - an integral internal diaphragm 11.21', which:
      - includes a through axial hole, and
      - defines, in said counter-cannula support 11.20, a first axial cavity 11.210, so-called rear cavity, having at least one inner side wall contiguous to said diaphragm 11.21', at a first axial end zone 11.21, and a second axial cavity 11.211, so-called front cavity, open towards the outside at a second axial end zone 11.22, opposite to said first axial end zone 11.21, of the counter-cannula support 11.20, and
   - a through hole 11.212, which:
      - is made at said rear end 11.21, and
      - has the axis aligned with the axis of said second hole 13.2 of said mounting body 13 and is configured to contain a second section of said first part of said first conductor 12 distal to said first electrically conductive end;
- branching said first conductor 12 outside said injection cavity of molten plastic material of said mould and outside said mould, by means of said second part of said first conductor 12 having said second electrically conductive end;
- closing said mould and carrying out the injection of molten plastic material into said injection cavity of molten plastic material of said mould, burying in the injected molten plastic material:
   - said mounting body 13 including, at said first axial cavity 11.210 of said counter-cannula support 11.20, said end part of said axial end 11.2a of said counter-cannula 11.2, said first section of said first part of said first conductor 12 and said fixed electrical contact between said first electrically conductive end of said first conductor 12 and said first axial end 11.31 of said thermocouple means 13;
   - said second section of said first part of the first conductor 12;
   - a further part of said axial end 11.2a of said counter-cannula 11.2 contiguous to said end part of said counter-cannula and sealed in said axial through hole of said diaphragm 11.21' of said counter-cannula support 11.20;
- causing the injected molten plastic material to harden and extracting said counter-cannula support 11.20 from said mould, wherein said mounting body 13, said axial end 11.2a of said counter-cannula 11.2 and said second section of said first part of the first conductor 12 are incorporated.

Furthermore, the process according to the invention is characterized by the step consisting in forming said mounting body 13 into two separate half-bodies 13.3, which are then juxtaposed and made to mate by means of respective opposite faces at a plane containing the axes of said first hole 13.1 and of said second hole 13.2 of the mounting body 13.

The process according to the invention is further characterized by the step consisting in providing said opposite faces of said two half-bodies 13.3 with respective recesses and corresponding mounting pins, which are made to cooperate to carry out the assembly of the mounting body 13 by positive and/or force coupling.

Also, the process according to the invention is characterized by the steps consisting in providing in each of said opposite faces of the half-bodies 13.1 a respective first channel and a respective second channel, in which said first channel extends over the entire length of the assembled body 13 and said second channel extends laterally and communicates with respect to said first channel, and in juxtaposing said two half-bodies 13.3 forming said first hole 13.1 and said second hole 13.2 of the mounting body 13 by overlapping the respective first and second channels.

It is also to be noted that the process according to the invention is characterized by the step consisting in forming, on an outer side surface of an end portion 11.12 of said cannula support 11.1, a linear arrangement of recesses 12.13, extended in axial direction and configured as notches of a graduated scale, and in forming in said counter-cannula support 11.20 an elastic finger 11.221, projecting with respect to said second end zone 11.22 of the counter-cannula support 11.20 and having an elastically flexible tip, configured for cooperating in an elastic snap with each recess of said arrangement of recesses 12.13 formed in said outer side surface of said cannula support 11.10.

As can be seen from the foregoing, the present invention achieves the objects set forth in the initial part of the present description in a simple and effective way.

## Claims

1. Electrostimulation device (10) with a double percutaneous coaxial needle, comprising:
- two tubular bodies (11.10, 11.20), in plastic material, hereinafter referred to respectively as cannula support (11.10) and counter-cannula support (11.20), wherein the cannula support (11.10) and the counter-cannula support (11.20) are telescopically connected with the possibility of relative axial sliding;
- a cannula needle (11.1) and a counter-cannula needle (11.2), hereinafter referred to respectively as cannula (11.1) and counter-cannula (11.2), axially perforated, in electrical conductive material, wherein the cannula (11.1) is supported fixed and coaxial with respect to the cannula support (11.10) and the counter-cannula (11.2) is supported fixed and coaxial with respect to the counter-cannula support (11.20) and wherein the counter-cannula (11.2) is inserted in a coaxial arrangement and is electrically insulated with respect to the cannula (11.1), with the possibility of relative axial sliding;
**characterized in that** the cannula support (11.10) is, at least partially, included within the counter-cannula support (11.20) with the possibility of relative axial sliding.

2. Electrostimulation device (10) according to claim 1, **characterized in that**:
- the cannula support (11.10) is configured, at a first axial end zone (11.11), so-called front end, as a bottom having an axial through hole, with respect to which an axial end of said cannula is fixed (11.1), the remainder of the cannula (11.1) extending beyond said front end (11.11) and towards the outside of the cannula support (11.10);
- the cannula (11.1) has a sharp tip (11.1'), distal with respect to said front end (11.11) of the cannula support (11.10) and suitable for skin puncture;
- the counter-cannula support (11.20) comprises an integral internal diaphragm (11.21') provided in proximity to a first axial end zone (11.21), so-called rear end, distal with respect to said front end (11.11) of the cannula support (11.10), wherein said internal diaphragm (11.21'):
- comprises a through axial hole and defines in said counter-cannula support (11.20):
- a first axial cavity (11.210), so-called rear cavity, open towards the outside at said rear end (11.21), wherein said rear cavity (11.210) has at least one internal side wall contiguous to said diaphragm (11.21'), and
- a second axial cavity (11.211), so-called front cavity, open towards the outside at a second axial end zone (11.22) of said counter-cannula support (11.20), axially opposite to said rear end (11.21), wherein said cannula support (11.10) is inserted into said front cavity (11.211), by means of a second axial end zone (11.12) of said cannula support (11.10), axially opposite to said front end (11.11), with the possibility of relative axial sliding together with the respective cannula (11.1);
- the counter-cannula (11.2):
- is fixed, in a coaxial arrangement, and with an intermediate portion, with respect to the axial hole of said internal diaphragm (11.21') of the counter-cannula support (11.20);
- extends into and beyond said front cavity (11.211) of said counter-cannula support (11.20);
- has a non-sharp tip (11.2'), at a distal end with respect to said diaphragm (11.21') of the counter-cannula support (11.20), arranged beyond said counter-cannula support (11.20) and configured to extend outwards and inwards with respect to the sharp tip (11.1') of the cannula (11.1);
- has an axial end (11.2a), proximal to said diaphragm (11.21') opposite said non-sharp tip (11.2'), extending beyond said diaphragm (11.21') in said rear cavity (11.210);
and **in that** it comprises:
- a mounting body (13) of said counter-cannula (11.2) with respect to said counter-cannula support (11.20), which is fixed in said rear cavity (11.210) of the counter-cannula support (11.20) and has at least one outer side wall facing towards and contiguous with respect to said at least one inner side wall of said rear cavity (11.210) of said counter-cannula support (11.20) and wherein said mounting body (13):
- is made of electrically insulating material, includes a first hole (13.1), which is axially aligned with said axial hole of the diaphragm (11.21') of the counter-cannula support (11.20), and comprises a second through hole (13.2), lateral with respect to said first hole (13.1) and extending between said first hole (13.1), on which the hole (13.2) is open, and said at least one outer side wall of said mounting body (13), on which the hole (13.2) itself is open;
- wherein the counter-cannula (11.2) has said axial end (11.2a) sealingly inserted in said first hole (13.1) of said mounting body (13);
- wherein a thermocouple means (11.3) is inserted and fixed in said counter-cannula (11.2) in an axial arrangement and has:
- a first electrically conductive axial end (11.3a), arranged inside said first hole (13.1) of the mounting body (13) and electrically connected with respect to a first electrically conductive end of a first insulated electric conductor (12);
- a second end, axially opposite to said first end (11.3a), arranged at said non-sharp tip (11.2') of the counter-cannula (11.2);
- wherein said first insulated electrical conductor (12) has an intermediate portion, contiguous to said first electrically conductive end, inserted in said second through hole (13.2) of the mounting body (13);
- wherein said counter-cannula support (11.20) comprises, in said rear end (11.21), a through hole (11.212), whose axis is axially aligned with the axis of said second hole (13.2) of said mounting body (13);
- wherein said first insulated electrical conductor (12) has a further portion inserted in said through hole (11.212) of the counter-cannula support (11.20), is branched outside the counter-cannula support (11.20) and is electrically connected, by means of a second electrically conductive end, with respect to an external electrical control circuit.

3. Electrostimulation device (10) according to claim 1 and/or 2, having a monopolar configuration, wherein said external electric control circuit comprises an electric pulse generator and a neutral dispersion plate, adapted to close the electric circuit between a negative pole and a positive pole, and wherein said cannula (11.1) is electrically insulated over its entire outer surface.

4. Electrostimulation device (10) according to claim 1 and/or 2, having a bipolar configuration, wherein said cannula (11.1) is electrically connected with respect to said external electrical control circuit by means of a second insulated electrical conductor (14), and wherein the cannula (11.1) has at least an electrically conductive end portion, while it is electrically insulated over the entire remaining part of its outer surface.

5. Electrostimulation device (10) according to any one of the preceding claims, **characterized in that** said mounting body (13) comprises two half-bodies (13.3), juxtaposed and mating at respective opposite faces in a plane containing the axes of said first hole (13.1) and said second hole (13.2) of the mounting body (13).

6. Electrostimulation device (10) according to claim 5, **characterized in that** said opposite faces of said two half-bodies (13.3) have respective recesses (13.41) and mounting pins (13.42), mutually complementary and opposite, which form the assembly of the mounting body (13) by means of positive and/or force coupling.

7. Electrostimulation device (10) according to claim 5 and/or 6, **characterized in that** each of said opposite faces of the half-bodies (13.3) has a respective first channel and a respective second channel, wherein said first channel is extended over the entire length of the assembled body (13) and said second channel extends laterally and communicates with respect to said first channel, so that said two juxtaposed and mutually fixed half-bodies (13.3) provide, by means of said respective first channel and second channel, reciprocally superimposed, said first hole (13.1) and said second hole (13.2) of the mounting body (13).

8. Electrostimulation device (10) according to any one of the preceding claims, **characterized in that** said cannula support (11.10) has, on an outer side surface, a linear arrangement of recesses (12.13), extending in the axial direction and configured as notches of a graduated scale, and **in that** said counter-cannula support (11.20) comprises an elastic finger (11.221), projecting with respect to said second end zone (11.22) of the counter-cannula support (11.20) and having an elastically flexible tip, configured for cooperating in an elastic snap with each recess of said arrangement of recesses (12.13) provided in said side surface of said cannula support (11.10).

9. Process for manufacturing an electrostimulation device (10) with a double percutaneous coaxial needle according to one of claims 1 to 7, wherein said device (10) comprises:
- two tubular bodies (11.10, 11.20), in plastic material, hereinafter referred to respectively as cannula support (11.10) and counter-cannula support (11.20), wherein the cannula support (11.10) and the counter-cannula support (11.20) are telescopically connected with the possibility of relative axial sliding;
- a cannula needle (11.1) and a counter-cannula needle (11.2), hereinafter referred to respectively as cannula (11.1) and counter-cannula (11.2), axially perforated, in electrical conductive material, wherein the cannula (11.1) is supported fixed and coaxial with respect to the cannula support (11.10) and the counter-cannula (11.2) is supported fixed and coaxial with respect to the counter-cannula support (11.20) and wherein the counter-cannula (11.2) is inserted in a coaxial arrangement and is electrically insulated with respect to the cannula (11.1), with the possibility of relative axial sliding;
**characterized in that** the cannula support (11.10) is movably inserted, at least partially, in the counter-cannula support (11.20) with the possibility of relative axial sliding.

10. Process according to claim 9, **characterized in that** it comprises the following production steps for producing said counter-cannula support (11.20) with relative counter-cannula (11.2) fixed with respect to said counter-cannula support (11.20):
- providing a counter-cannula (11.2), which has a first axial end (11.2a) opposite a second axial end, so-called non-sharp tip (11.2');
- providing a thermocouple means (11.3), configured to be inserted and fixed in said counter-cannula (11.2) in an axial arrangement and having a first axial end (11.31), electrically conductive, and a second axial end opposite to said first end (11.31);
- inserting and fixing said thermocouple means (11.3) in an axial arrangement in said counter-cannula (11.2), placing said first axial end (11.31) of said thermocouple means (11.3) in proximity to said first axial end (11.2a) of the counter-cannula (11.2) and said second axial end of said thermocouple means (11.3) in proximity to said non-sharp tip;
- providing a first insulated electrical conductor (12), hereinafter referred to as the first conductor (12), which has a first electrically conductive end and a second electrically conductive end;
- making a mounting body (13) of said counter-cannula (11.2) with respect to said counter-cannula support (11.20), wherein said mounting body (13) is made of electrically insulating material and is provided with:
- at least one outer side wall;
- a first hole (13.1), configured to house a first part, the so-called end part, of said axial end (11.2a) of the counter-cannula (11.2),
- a second hole (13.2), passing through and extending between said first hole (13.1) and said at least one outer side wall of said mounting body (13), wherein said second hole (13.2) is configured to house a first section of a first part of said first conductor (12), wherein said first section is contiguous to said first electrically conductive end of said first conductor (12), which is arranged outside said second hole (13.2);
- inserting said end part of said axial end (11.2a) of the counter-cannula (11.2) in said first hole (13.1) of said mounting body (13), inserting said first section of said first part of said first conductor (12) in said second hole (13.2) of said mounting body (13) and juxtaposing said first electrically conductive end of said first conductor (12) and said first axial end (11.31) of said thermocouple means (11.3), making a fixed electrical contact;
- providing a mould configured for injection moulding of molten plastic material and metal and providing in said mould moulding means for said counter-cannula support (11.20), wherein said moulding means are configured as injection cavities of molten plastic material;
- arranging in said injection cavity of molten plastic material said mounting body (13) including said end part of said axial end (11.2a) of said counter-cannula (11.2), said first section of said first part of said first conductor (12) and said fixed electrical contact between said first electrically conductive end of said first conductor (12) and said first axial end (11.31) of said thermocouple means (13);
- configuring said injection cavity of molten plastic material to form said counter-cannula support (11.20) as a tubular body and to form in said counter-cannula support (11.20):
- an integral internal diaphragm (11.21'), which:
- includes a through axial hole, and
- defines, in said counter-cannula support (11.20), a first axial cavity (11.210), so-called rear cavity, having at least one inner side wall contiguous to said diaphragm (11.21'), at a first axial end zone (11.21), and a second axial cavity (11.211), so-called front cavity, open towards the outside at a second axial end zone (11.22), opposite to said first axial end zone (11.21), of the counter-cannula support (11.20), and
- a through hole (11.212), which:
- is made at said rear end (11.21), and
- has the axis aligned with the axis of said second hole (13.2) of said mounting body (13) and is configured to contain a second section of said first part of said first conductor (12) distal to said first electrically conductive end;
- branching said first conductor (12) outside said injection cavity of molten plastic material of said mould and outside said mould, by means of said second part of said first conductor (12) having said second electrically conductive end;
- closing said mould and carrying out the injection of molten plastic material into said injection cavity of molten plastic material of said mould, burying in the injected molten plastic material:
- said mounting body (13) including, at said first axial cavity (11.210) of said counter-cannula support (11.20), said end part of said axial end (11.2a) of said counter-cannula (11.2), said first section of said first part of said first conductor (12) and said fixed electrical contact between said first electrically conductive end of said first conductor (12) and said first axial end (11.31) of said thermocouple means (13);
- said second section of said first part of the first conductor (12);
- a further part of said axial end (11.2a) of said counter-cannula (11.2) contiguous to said end part of said counter-cannula and sealed in said axial through hole of said diaphragm (11.21') of said counter-cannula support (11.20);
- causing the injected molten plastic material to harden and extracting said counter-cannula support (11.20) from said mould, wherein said mounting body (13), said axial end (11.2a) of said counter-cannula (11.2) and said second section of said first part of the first conductor (12) are incorporated.

11. Process according to claim 10 for the production of the device (10) according to claim 5, **characterized by** the step consisting in forming said mounting body (13) into two separate half-bodies (13.3), which are then juxtaposed and made to mate by means of respective opposite faces at a plane containing the axes of said first hole (13.1) and of said second hole (13.2) of the mounting body (13).

12. Process according to claim 10 for the production of the device (10) according to claim 6, **characterized by** the step consisting in providing said opposite faces of said two half-bodies (13.3) with respective recesses (13.41) and corresponding mounting pins (13.42), which are made to cooperate to carry out the assembly of the mounting body (13) by positive and/or force coupling.

13. Process according to claim 10 for the production of the device (10) according to claim 7, **characterized by** the steps consisting in providing in each of said opposite faces of the half-bodies (13.1) a respective first channel and a respective second channel, in which said first channel extends over the entire length of the assembled body (13) and said second channel extends laterally and communicates with respect to said first channel, and in juxtaposing said two half-bodies (13.3) forming said first hole (13.1) and said second hole (13.2) of the mounting body (13) by overlapping the respective first and second channels.

14. Process according to claim 10 for the production of the device (10) according to claim 8, **characterized by** the step consisting in forming, on an outer side surface of an end portion (11.12) of said cannula support (11.1), a linear arrangement of recesses (12.13), extended in axial direction and configured as notches of a graduated scale, and in forming in said counter-cannula support (11.20) an elastic finger (11.221), projecting with respect to said second end zone (11.22) of the counter-cannula support (11.20) and having an elastically flexible tip, configured for cooperating in an elastic snap with each recess of said arrangement of recesses (12.13) formed in said outer side surface of said cannula support (11.10).

## Patentansprüche

1. Vorrichtung (10) zur Elektrostimulation mit koaxialer, perkutaner Doppelnadel, umfassend:
- zwei Röhrenkörper (11.10, 11.20) aus Kunststoff, nachfolgend jeweils als Kanülenhalter (11.10) und Gegenkanülenhalter (11.20) bezeichnet, wobei der Kanülenhalter (11.10) und der Gegenkanülenhalter (11.20) teleskopartig miteinander verbunden sind, mit der Möglichkeit einer relativen axialen Verschiebung,
- eine Kanülennadel (11.1) und eine Gegenkanülennadel (11.2), nachfolgend jeweils als Kanüle (11.1) und Gegenkanüle (11.2) bezeichnet, axial durchbohrt, aus elektrisch leitfähigem Material, wobei die Kanüle (11.1) fest und koaxial relativ zum Kanülenhalter (11.10) gehalten ist und die Gegenkanüle (11.2) fest und koaxial relativ zum Gegenkanülenhalter (11.20) gehalten ist, und wobei die Gegenkanüle (11.2) koaxial angeordnet ist und elektrisch von der Kanüle (11.1) isoliert ist, mit der Möglichkeit einer relativen axialen Verschiebung;
durch **dadurch gekennzeichnet, dass** der Kanülenhalter (11.10) zumindest teilweise innerhalb des Gegenkanülenhalters (11.20) aufgenommen ist, mit der Möglichkeit einer relativen axialen Verschiebung.

2. Vorrichtung (10) zur Elektrostimulation nach Anspruch 1, **dadurch gekennzeichnet, dass**:
- der Kanülenhalter (11.10) an einem ersten axialen Endbereich (11.11), dem sogenannten vorderen Ende, als Boden mit einer durchgehenden axialen Öffnung ausgebildet ist, an der ein axiales Ende der genannten Kanüle (11.1) befestigt ist, wobei der restliche Teil der Kanüle (11.1) über das genannte vordere Ende (11.11) hinaus und nach außen aus dem Kanülenhalter (11.10) herausragt;
- die Kanüle (11.1) eine scharfe Spitze (11.1') aufweist, distal zu dem genannten vorderen Ende (11.11) des Kanülenhalters (11.10) und geeignet zum Hautdurchstich;
- der Gegenkanülenhalter (11.20) eine integrale innere Membran (11.21') umfasst, die in der Nähe eines ersten axialen Endbereichs (11.21), dem sogenannten hinteren Ende, angeordnet ist, distal zu dem genannten vorderen Ende (11.11) des Kanülenhalters (11.10), wobei die genannte innere Membran (11.21') Folgendes aufweist:
- eine durchgehende axiale Öffnung umfasst und im genannten Gegenkanülenhalter (11.20) Folgendes begrenzt:
- eine erste axiale Kavität (11.210), sogenannte hintere Kavität, die an dem genannten hinteren Ende (11.21) nach außen geöffnet ist, wobei die genannte hintere Kavität (11.210) mindestens eine innere Seitenwand aufweist, die an die genannte Membran (11.21') angrenzt, und
- eine zweite axiale Kavität (11.211), sogenannte vordere Kavität, die an einem zweiten axialen Endbereich (11.22) des genannten Gegenkanülenhalters (11.20), axial entgegengesetzt zu dem genannten hinteren Ende (11.21), nach außen geöffnet ist, wobei in die genannte vordere Kavität (11.211) der genannte Kanülenhalter (11.10) mittels eines zweiten axialen Endbereichs (11.12) des genannten Kanülenhalters (11.10), axial entgegengesetzt zu dem genannten vorderen Ende (11.11), eingesetzt ist, mit der Möglichkeit einer relativen axialen Verschiebung zusammen mit der entsprechenden Kanüle (11.1);
- die Gegenkanüle (11.2):
- koaxial befestigt ist, mit einem mittleren Abschnitt, relativ zu der axialen Öffnung der genannten inneren Membran (11.21') des Gegenkanülenhalters (11.20);
- sich innerhalb und über die genannte vordere Kavität (11.211) des genannten Gegenkanülenhalters (11.20) hinaus erstreckt;
- eine stumpfe Spitze (11.2') an einem distalen Ende relativ zu der genannten Membran (11.21') des Gegenkanülenhalters (11.20) aufweist, die über den genannten Gegenkanülenhalter (11.20) hinausragt und so konfiguriert ist, dass sie sich außerhalb und innerhalb relativ zur scharfen Spitze (11.1') der Kanüle (11.1) erstreckt;
- ein axiales Ende (11.2a) aufweist, proximal zu der genannten Membran (11.21'), gegenüberliegend zu der genannten stumpfen Spitze (11.2'), das über die genannte Membran (11.21') hinaus in der genannten hinteren Kavität (11.210) angeordnet ist;
und dadurch, dass es Folgendes umfasst:
- einen Montagekörper (13) der genannten Gegenkanüle (11.2) relativ zu dem genannten Gegenkanülenhalter (11.20), der in der genannten hinteren Kavität (11.210) des Gegenkanülenhalters (11.20) befestigt ist und mindestens eine äußere Seitenwand aufweist, die der genannten mindestens einen inneren Seitenwand der genannten hinteren Kavität (11.210) des genannten Gegenkanülenhalters (11.20) zugewandt und angrenzend ist, wobei der genannte Montagekörper (13):
- aus elektrisch isolierendem Material besteht, eine erste Bohrung (13.1) umfasst, die axial mit der genannten axialen Öffnung der Membran (11.21') des Gegenkanülenhalters (11.20) ausgerichtet ist, und eine zweite durchgehende Bohrung (13.2) umfasst, die seitlich zu der genannten ersten Bohrung (13.1) angeordnet ist und sich zwischen der genannten ersten Bohrung (13.1), auf die die Bohrung (13.2) geöffnet ist, und der genannten mindestens einen äußeren Seitenwand des genannten Montagekörpers (13), auf die die Bohrung (13.2) ebenfalls geöffnet ist, erstreckt;
- wobei die Gegenkanüle (11.2) das genannte axiale Ende (11.2a) aufweist, das dicht in die genannte erste Bohrung (13.1) des genannten Montagekörpers (13) eingesetzt ist;
- wobei ein Thermoelementmittel (11.3) in die genannte Gegenkanüle (11.2) axial eingesetzt und fixiert ist und Folgendes aufweist:
- ein erstes axiales Ende (11.3a), elektrisch leitfähig, das innerhalb der genannten ersten Bohrung (13.1) des Montagekörpers (13) angeordnet ist und elektrisch mit einem ersten elektrisch leitfähigen Ende eines ersten isolierten elektrischen Leiters (12) verbunden ist;
- ein zweites Ende, axial entgegengesetzt zu dem genannten ersten Ende (11.3a), das in Höhe der genannten stumpfen Spitze (11.2') der Gegenkanüle (11.2) angeordnet ist;
- wobei der genannte erste isolierte elektrische Leiter (12) einen mittleren Abschnitt aufweist, der an das genannte erste elektrisch leitfähige Ende angrenzt, der in die genannte zweite durchgehende Bohrung (13.2) des Montagekörpers (13) eingeführt ist;
- wobei der genannte Gegenkanülenhalter (11.20) in dem genannten hinteren Ende (11.21) eine durchgehende Öffnung (11.212) umfasst, deren Achse axial mit der Achse der genannten zweiten Bohrung (13.2) des genannten Montagekörpers (13) ausgerichtet ist;
- wobei der genannte erste isolierte elektrische Leiter (12) einen weiteren Abschnitt umfasst, der in die genannte durchgehende Öffnung (11.212) des Gegenkanülenhalters (11.20) eingesetzt ist, aus dem Gegenkanülenhalter (11.20) herausgeführt ist und elektrisch verbunden ist, mittels eines zweiten elektrisch leitfähigen Endes, mit einer externen elektrischen Steuerungsschaltung.

3. Vorrichtung (10) zur Elektrostimulation nach Anspruch 1 und/oder 2, wobei eine monopolare Konfiguration vorliegt, wobei die genannte externe elektrische Steuerungsschaltung einen elektrischen Impulsgenerator und eine neutrale Ableitplatte umfasst, die dazu geeignet ist, den elektrischen Kreis zwischen einem negativen und einem positiven Pol zu schließen, und wobei die genannte Kanüle (11.1) auf ihrer gesamten äußeren Oberfläche elektrisch isoliert ist.

4. Vorrichtung (10) zur Elektrostimulation nach Anspruch 1 und/oder 2, wobei eine bipolare Konfiguration vorliegt, wobei die genannte Kanüle (11.1) elektrisch mit der genannten externen elektrischen Steuerungsschaltung mittels eines zweiten isolierten elektrischen Leiters (14) verbunden ist, und wobei die Kanüle (11.1) mindestens einen elektrisch leitfähigen Endabschnitt aufweist, während der restliche Teil ihrer äußeren Oberfläche elektrisch isoliert ist.

5. Vorrichtung (10) zur Elektrostimulation nach einem der vorhergehenden Ansprüche, durch **dadurch gekennzeichnet, dass** der genannte Montagekörper (13) zwei Halbkörper (13.3) umfasst, die an entsprechenden gegenüberliegenden Flächen in einer Ebene aneinandergefügt sind, die die Achsen der genannten ersten Bohrung (13.1) und der genannten zweiten Bohrung (13.2) des Montagekörpers (13) enthält.

6. Vorrichtung (10) zur Elektrostimulation nach Anspruch 5, **dadurch gekennzeichnet, dass** die genannten gegenüberliegenden Flächen der genannten beiden Halbkörper (13.3) jeweilige Vertiefungen (13.41) und Montagezapfen (13.42) aufweisen, die zueinander komplementär und gegenüberliegend sind und die Montage des genannten Montagekörpers (13) mittels geometrischer und/oder kraftschlüssiger Verbindung ermöglichen.

7. Vorrichtung (10) zur Elektrostimulation nach Anspruch 5 und/oder 6, **dadurch gekennzeichnet, dass** jede der genannten gegenüberliegenden Flächen der Halbkörper (13.3) jeweils einen ersten Kanal und einen zweiten Kanal aufweist, wobei der genannte erste Kanal über die gesamte Länge des zusammengesetzten Körpers (13) verläuft und der genannte zweite Kanal seitlich verläuft und mit dem genannten ersten Kanal in Verbindung steht, sodass die genannten beiden Halbkörper (13.3), aneinandergefügt und miteinander verbunden, mittels der genannten jeweiligen überlagerten ersten und zweiten Kanäle die genannte erste Bohrung (13.1) und die genannte zweite Bohrung (13.2) des genannten Montagekörpers (13) bilden.

8. Vorrichtung (10) zur Elektrostimulation nach einem der vorhergehenden Ansprüche, durch **dadurch gekennzeichnet, dass** der genannte Kanülenhalter (11.10) auf einer äußeren Seitenfläche eine lineare Anordnung von Vertiefungen (12.13) aufweist, die sich in axialer Richtung erstrecken und als Markierungen einer Skala ausgebildet sind, und dass der genannte Gegenkanülenhalter (11.20) einen elastischen Finger (11.221) umfasst, der sich von dem genannten zweiten Endbereich (11.22) des Gegenkanülenhalters (11.20) absetzt und eine elastisch biegbare Spitze aufweist, die so ausgebildet ist, dass sie federnd mit jeder Vertiefung der genannten Anordnung von Vertiefungen (12.13) an der genannten äußeren Seitenfläche des genannten Kanülenhalters (11.10) zusammenwirkt.

9. Verfahren zur Herstellung einer Vorrichtung (10) zur Elektrostimulation mit koaxialer perkutaner Doppelnadel nach einem der Ansprüche 1 bis 7, wobei die genannte Vorrichtung (10) Folgendes umfasst:
- zwei Röhrenkörper (11.10, 11.20) aus Kunststoff, nachfolgend jeweils als Kanülenhalter (11.10) und Gegenkanülenhalter (11.20) bezeichnet, wobei der Kanülenhalter (11.10) und der Gegenkanülenhalter (11.20) teleskopartig miteinander verbunden sind, mit der Möglichkeit einer relativen axialen Verschiebung;
- eine Kanülennadel (11.1) und eine Gegenkanülennadel (11.2), nachfolgend jeweils als Kanüle (11.1) und Gegenkanüle (11.2) bezeichnet, axial durchbohrt, aus elektrisch leitfähigem Material, wobei die Kanüle (11.1) fest und koaxial relativ zum Kanülenhalter (11.10) gehalten ist und die Gegenkanüle (11.2) fest und koaxial relativ zum Gegenkanülenhalter (11.20) gehalten ist, und wobei die Gegenkanüle (11.2) koaxial angeordnet ist und elektrisch von der Kanüle (11.1) isoliert ist, mit der Möglichkeit einer relativen axialen Verschiebung,
durch **dadurch gekennzeichnet, dass** der Kanülenhalter (11.10) mindestens teilweise innerhalb des Gegenkanülenhalters (11.20) beweglich mit axialer Relativverschiebung eingesetzt ist.

10. Verfahren nach Anspruch 9, **dadurch gekennzeichnet, dass** es die folgenden Schritte zur Herstellung des genannten Gegenkanülenhalters (11.20) mit der zugehörigen Gegenkanüle (11.2), die relativ zu dem genannten Gegenkanülenhalters (11.20) befestigt ist, umfasst:
- Bereitstellen einer Gegenkanüle (11.2), die ein erstes axiales Ende (11.2a) aufweist, das einem zweiten axialen Ende, genannt Spitze, gegenüberliegt, wobei die Spitze nicht scharf ist (11.2');
- Bereitstellen eines Thermoelements (11.3), das so konfiguriert ist, dass es axial in die genannte Gegenkanüle (11.2) eingeführt und darin fixiert werden kann, wobei das Thermoelement (11.3) ein erstes axiales Ende (11.31) aufweist, das elektrisch leitfähig ist, und ein zweites axiales Ende, das dem genannten ersten Ende (11.31) gegenüberliegt;
- Einsetzen und Befestigen des genannten Thermoelements (11.3) axial in der genannten Gegenkanüle (11.2), wobei das erste axiale Ende (11.31) des genannten Thermoelements (11.3) in der Nähe des genannten ersten axialen Endes (11.2a) der Gegenkanüle (11.2) angeordnet wird und das zweite axiale Ende des genannten Thermoelements (11.3) in der Nähe der genannten stumpfen Spitze (11.2') positioniert wird;
- Bereitstellen eines ersten isolierten elektrischen Leiters (12), nachfolgend als erster Leiter (12) bezeichnet, der ein erstes elektrisch leitfähiges Ende und ein zweites elektrisch leitfähiges Ende aufweist;
- Herstellen eines Montagekörpers (13) für die genannte Gegenkanüle (11.2) relativ zu dem genannten Gegenkanülenhalter (11.20), wobei der Montagekörper (13) aus elektrisch isolierendem Material gefertigt ist und Folgendes umfasst:
- mindestens eine äußere Seitenwand;
- eine erste Bohrung (13.1), die so konfiguriert ist, dass sie einen ersten Abschnitt, genannt Endabschnitt, des genannten axialen Endes (11.2a) der Gegenkanüle (11.2) aufnehmen kann;
- eine zweite Bohrung (13.2), die durchgehend ist und sich zwischen der genannten ersten Bohrung (13.1) und der genannten mindestens einen äußeren Seitenwand des genannten Montagekörpers (13) erstreckt, wobei die genannte zweite Bohrung (13.2) so konfiguriert ist, dass sie einen ersten Abschnitt eines ersten Teils des genannten ersten Leiters (12) aufnehmen kann, wobei der genannte erste Abschnitt an das genannte erste elektrisch leitfähige Ende des genannten ersten Leiters (12) angrenzt, das außerhalb der genannten zweiten Bohrung (13.2) angeordnet ist;
- Einsetzen des genannten Endabschnitts des genannten axialen Endes (11.2a) der Gegenkanüle (11.2) in die genannte erste Bohrung (13.1) des genannten Montagekörpers (13), Einführen des genannten ersten Abschnitts des genannten ersten Teils des genannten ersten Leiters (12) in die genannte zweite Bohrung (13.2) des genannten Montagekörpers (13) und Aneinanderlegen des genannten ersten elektrisch leitfähigen Endes des genannten ersten Leiters (12) und des genannten ersten axialen Endes (11.31) des genannten Thermoelements (11.3), um einen festen elektrischen Kontakt herzustellen;
- Bereitstellen einer Form, die für das Spritzgießen von geschmolzenem Kunststoff und Metall konfiguriert ist, und Bereitstellen von Formmitteln in der genannten Form zur Ausbildung des genannten Gegenkanülenhalters (11.20), wobei die genannten Formmittel als Hohlräume zum Einspritzen von geschmolzenem Kunststoff ausgebildet sind;
- Anordnen des genannten Montagekörpers (13), einschließlich des genannten Endabschnitts des genannten axialen Endes (11.2a) der genannten Gegenkanüle (11.2), des genannten ersten Abschnitts des genannten ersten Teils des genannten ersten Leiters (12) und des genannten festen elektrischen Kontakts zwischen dem genannten ersten elektrisch leitfähigen Ende des genannten ersten Leiters (12) und dem genannten ersten axialen Ende (11.31) des genannten Thermoelements (11.3), in dem genannten Hohlraum für das Einspritzen von geschmolzenem Kunststoff;
- Konfigurieren des genannten Hohlraums für das Einspritzen von geschmolzenem Kunststoff zur Bildung des genannten Gegenkanülenhalters (11.20) als Röhrenkörper und zur Ausbildung in dem genannten Gegenkanülenhalter (11.20):
- einer integralen inneren Membran (11.21'), die:
- eine durchgehende axiale Öffnung umfasst und
- eine erste axiale Kavität (11.210), sogenannte hintere Kavität, innerhalb des genannten Gegenkanülenhalters (11.20) begrenzt, die mindestens eine innere Seitenwand aufweist, die an die genannte Membran (11.21') angrenzt, an einem ersten axialen Endbereich (11.21), sowie eine zweite axiale Kavität (11.211), genannt vordere Kavität, die an einem zweiten axialen Endbereich (11.22), gegenüberliegend zu dem genannten ersten axialen Endbereich (11.21), nach außen geöffnet ist;
- eine durchgehende Öffnung (11.212), die:
- in der Nähe des genannten hinteren Endes (11.21) ausgebildet ist und
- eine Achse aufweist, die mit der Achse der genannten zweiten Bohrung (13.2) des genannten Montagekörpers (13) ausgerichtet ist, und so konfiguriert ist, dass sie einen zweiten Abschnitt des genannten ersten Teils des genannten ersten Leiters (12), der distal zum genannten ersten elektrisch leitfähigen Ende liegt, aufnehmen kann;
- Herausführen des genannten ersten Leiters (12) außerhalb des genannten Hohlraums für das Einspritzen von geschmolzenem Kunststoff der genannten Form und außerhalb der genannten Form, mittels des genannten zweiten Teils des genannten ersten Leiters (12), das das genannte zweite elektrisch leitfähige Ende aufweist;
- Schließen der genannten Form und Einspritzen von geschmolzenem Kunststoff in den genannten Hohlraum der genannten Form, wodurch in dem eingespritzten geschmolzenen Kunststoff eingebettet werden:
- der genannte Montagekörper (13), einschließlich in der genannten hinteren Kavität (11.210) des genannten Gegenkanülenhalters (11.20) des genannten Endabschnitts des genannten axialen Endes (11.2a) der genannten Gegenkanüle (11.2), des genannten ersten Abschnitts des genannten ersten Teils des genannten ersten Leiters (12) und des genannten festen elektrischen Kontakts zwischen dem genannten ersten elektrisch leitfähigen Ende des genannten ersten Leiters (12) und dem genannten ersten axialen Ende (11.31) des genannten Thermoelements (11.3);
- der genannte zweite Abschnitt des genannten ersten Teils des genannten ersten Leiters (12);
- ein weiterer Teil des genannten axialen Endes (11.2a) der genannten Gegenkanüle (11.2), der an den genannten Endabschnitt der genannten Gegenkanüle angrenzt und dicht in der genannten durchgehenden axialen Öffnung der genannten Membran (11.21') des genannten Gegenkanülenhalters (11.20) versiegelt ist;
- Aushärten des eingespritzten geschmolzenen Kunststoffs und Entnahme des genannten Gegenkanülenhalters (11.20) aus der genannten Form, in dem der genannte Montagekörper (13), das genannte axiale Ende (11.2a) der genannten Gegenkanüle (11.2) und der genannte zweite Abschnitt des genannten ersten Teils des genannten ersten Leiters (12) eingebettet und fixiert sind.

11. Verfahren nach Anspruch 10 zur Herstellung der Vorrichtung (10) nach Anspruch 5, durch **dadurch gekennzeichnet, dass** der genannte Montagekörper (13) aus zwei separaten Halbkörpern (13.3) besteht, die dann aneinandergelegt und an gegenüberliegenden Flächen aneinandergefügt werden auf einer Ebene, die die Achsen der genannten ersten Bohrung (13.1) und der genannten zweiten Bohrung (13.2) des genannten Montagekörpers (13) enthält.

12. Verfahren nach Anspruch 10 zur Herstellung der Vorrichtung (10) nach Anspruch 6, durch **dadurch gekennzeichnet, dass** die genannten gegenüberliegenden Flächen der genannten beiden Halbkörper (13.3) jeweilige Vertiefungen (13.41) und zugehörige Montagezapfen (13.42) aufweisen, die zusammenwirken, um die Montage des genannten Montagekörpers (13) mittels gegenseitigem geometrischem und/oder kraftschlüssigem Eingriff zu ermöglichen.

13. Verfahren nach Anspruch 10 zur Herstellung der Vorrichtung (10) nach Anspruch 7, durch **dadurch gekennzeichnet, dass** in jeder der genannten gegenüberliegenden Flächen der Halbkörper (13.3) jeweils ein erster Kanal und ein zweiter Kanal vorgesehen sind, wobei der genannte erste Kanal über die gesamte Länge des zusammengesetzten Körpers (13) verläuft und der genannte zweite Kanal seitlich verläuft und mit dem genannten ersten Kanal in Verbindung steht, und dass die genannten beiden Halbkörper (13.3) aneinandergelegt werden, wodurch die genannte erste Bohrung (13.1) und die genannte zweite Bohrung (13.2) des genannten Montagekörpers (13) durch Überlagerung der genannten jeweiligen ersten und zweiten Kanäle gebildet werden.

14. Verfahren nach Anspruch 10 zur Herstellung der Vorrichtung (10) nach Anspruch 8, durch **dadurch gekennzeichnet, dass** auf einer äußeren Seitenfläche eines Endabschnitts (11.12) des genannten Kanülenhalters (11.10) eine lineare Anordnung von Vertiefungen (12.13) vorgesehen ist, die sich in axialer Richtung erstrecken und als Markierungen einer Skala ausgebildet sind, und dass in dem genannten Gegenkanülenhalters (11.20) ein elastischer Finger (11.221) vorgesehen ist, der sich von dem genannten zweiten Endbereich (11.22) des genannten Gegenkanülenhalters (11.20) absetzt und eine elastisch biegbare Spitze aufweist, die so konfiguriert ist, dass sie federnd mit jeder Vertiefung der genannten Anordnung von Vertiefungen (12.13) an der genannten äußeren Seitenfläche des genannten Kanülenhalters (11.10) zusammenwirkt.

## Revendications

1. Dispositif (10) d'électrostimulation à double aiguille percutanée coaxiale, comprenant :
- deux corps tubulaires (11.10, 11.20), en matière plastique, ci-après dénommés respectivement support de canule (11.10) et support de contre-canule (11.20), dans lequel le support de canule (11.10) et le support de contre-canule (11.20) sont raccordés de manière télescopique avec possibilité de coulissement axial relatif,
- une aiguille à canule (11.1) et une aiguille à contre-canule (11.2), ci-après dénommées respectivement canule (11.1) et contre-canule (11.2), percées axialement, en matériau conducteur électrique, dans lequel la canule (11.1) est supportée fixe et coaxiale par rapport au support de canule (11.10) et la contre-canule (11.2) est supportée fixe et coaxiale par rapport au support de contre-canule (11.20), et dans lequel la contre-canule (11.2) est insérée en disposition coaxiale et est isolée électriquement par rapport à la canule (11.1), avec possibilité de coulissement axial relatif ;
**caractérisé en ce que** le support de canule (11.10) est au moins partiellement inclus dans le support de contre-canule (11.20), avec possibilité de coulissement axial relatif.

2. Dispositif (10) d'électrostimulation selon la revendication 1, **caractérisé en ce que** :
- le support de canule (11.10) est configuré, au niveau d'une première zone d'extrémité axiale (11.11), dite extrémité antérieure, comme un fond présentant un trou axial traversant, par rapport auquel une extrémité axiale de ladite canule (11.1) est fixée, la partie restante de la canule (11.1) étant étendue au-delà de ladite extrémité antérieure (11.11) et vers l'extérieur du support de canule (11.10) ;
- la canule (11.1) présente une pointe tranchante (11.1'), distale par rapport à ladite extrémité antérieure (11.11) du support de canule (11.10) et apte à la ponction cutanée;
- le support de contre-canule (11.20) comprend un diaphragme interne intégral (11.21') pourvu à proximité d'une première zone d'extrémité axiale (11.21), dite extrémité postérieure, distale par rapport à ladite extrémité antérieure (11.11) du support de canule (11.10), dans lequel ledit diaphragme interne (11.21') :
- comprend un trou axial traversant et délimite dans ledit support de contre-canule (11.20) :
- une première cavité axiale (11.210), dite cavité postérieure, ouverte vers l'extérieur au niveau de ladite extrémité postérieure (11.21), dans laquelle ladite cavité postérieure (11.210) présente au moins une paroi latérale interne contiguë audit diaphragme (11.21'), et
- une seconde cavité axiale (11.211), dite cavité antérieure, ouverte vers l'extérieur au niveau d'une seconde zone d'extrémité axiale (11.22) dudit support de contre-canule (11.20), axialement opposée à ladite extrémité postérieure (11.21), dans laquelle dans ladite cavité antérieure (11.211) est inséré ledit support de canule (11.10), au moyen d'une seconde zone d'extrémité axiale (11.12) dudit support de canule (11.10), axialement opposée à ladite extrémité antérieure (11.11), avec possibilité de coulissement axial relatif avec la canule correspondante (11.1) ;
- la contre-canule (11.2) :
- est fixée, en disposition coaxiale, et avec une portion intermédiaire, par rapport au trou axial dudit diaphragme interne (11.21') du support de contre-canule (11.20) ;
- s'étend à l'intérieur et au-delà de ladite cavité antérieure (11.211) dudit support de contre-canule (11.20) ;
- présente une pointe non tranchante (11.2'), à une extrémité distale par rapport audit diaphragme (11.21') du support de contre-canule (11.20), disposée au-delà dudit support de contre-canule (11.20) et configurée pour s'étendre à l'extérieur et à l'intérieur par rapport à la pointe tranchante (11.1') de la canule (11.1) ;
- présente une extrémité axiale (11.2a), proximale par rapport audit diaphragme (11.21') opposée à ladite pointe non tranchante (11.2'), étendue au-delà dudit diaphragme (11.21') dans ladite cavité postérieure (11.210) ;
et **en ce qu'**il comprend :
- un corps de montage (13) de ladite contre-canule (11.2) par rapport audit support de contre-canule (11.20), qui est fixé dans ladite cavité postérieure (11.210) du support de contre-canule (11.20) et présente au moins une paroi latérale externe orientée vers et contiguë à ladite au moins une paroi latérale interne de ladite cavité postérieure (11.210) dudit support de contre-canule (11.20), et dans lequel ledit corps de montage (13) :
- est réalisé en matériau électriquement isolant, comprend un premier trou (13.1), aligné axialement avec ledit trou axial du diaphragme (11.21') du support de contre-canule (11.20), et comprend un second trou traversant (13.2), latéral par rapport audit premier trou (13.1) et étendu entre ledit premier trou (13.1), sur lequel le trou (13.2) est ouvert, et ladite au moins une paroi latérale externe dudit corps de montage (13), sur laquelle le trou (13.2) est également ouvert ;
- dans lequel la contre-canule (11.2) présente ladite extrémité axiale (11.2a) insérée de manière étanche dans ledit premier trou (13.1) dudit corps de montage (13) ;
- dans lequel un moyen à thermocouple (11.3) est inséré et fixé dans ladite contre-canule (11.2) en disposition axiale et présente :
- une première extrémité axiale (11.3a) électriquement conductrice, disposée à l'intérieur dudit premier trou (13.1) du corps de montage (13) et électriquement reliée à une première extrémité électriquement conductrice d'un premier conducteur électrique isolé (12) ;
- une seconde extrémité, axialement opposée à ladite première extrémité (11.3a), disposée au niveau de ladite pointe non tranchante (11.2') de la contre-canule (11.2) ;
- dans lequel ledit premier conducteur électrique isolé (12) présente une portion intermédiaire, contiguë à ladite première extrémité électriquement conductrice, insérée dans ledit second trou traversant (13.2) du corps de montage (13) ;
- dans lequel ledit support de contre-canule (11.20) comprend, dans ladite extrémité postérieure (11.21), un trou traversant (11.212), dont l'axe est aligné axialement avec l'axe dudit second trou (13.2) dudit corps de montage (13) ;
- dans lequel ledit premier conducteur électrique isolé (12) présente une autre portion insérée dans ledit trou traversant (11.212) du support de contre-canule (11.20), est dérivé à l'extérieur du support de contre-canule (11.20) et est électriquement relié, au moyen d'une seconde extrémité électriquement conductrice, par rapport à un circuit électrique externe de commande.

3. Dispositif (10) d'électrostimulation selon la revendication 1 et/ou 2, présentant une configuration monopolaire, dans lequel ledit circuit électrique externe de commande comprend un générateur d'impulsions électriques et une plaque neutre de dispersion, apte à fermer le circuit électrique entre un pôle négatif et un pôle positif, et dans lequel ladite canule (11.1) est électriquement isolée sur toute sa surface externe.

4. Dispositif (10) d'électrostimulation selon la revendication 1 et/ou 2, présentant une configuration bipolaire, dans lequel ladite canule (11.1) est électriquement reliée audit circuit électrique externe de commande au moyen d'un deuxième conducteur électrique isolé (14), et dans lequel la canule (11.1) présente au moins une portion d'extrémité électriquement conductrice, tandis qu'elle est électriquement isolée sur toute la partie restante de sa surface externe.

5. Dispositif (10) d'électrostimulation selon l'une quelconque des revendications précédentes, **caractérisé en ce que** ledit corps de montage (13) comprend deux demi-corps (13.3), juxtaposés et accouplés au niveau de faces opposées respectives dans un plan contenant les axes dudit premier trou (13.1) et dudit second trou (13.2) du corps de montage (13).

6. Dispositif (10) d'électrostimulation selon la revendication 5, **caractérisé en ce que** lesdites faces opposées desdits deux demi-corps (13.3) présentent des évidements respectifs (13.41) et des broches de montage (13.42), réciproquement complémentaires et opposés, qui réalisent l'assemblage du corps de montage (13) par accouplement géométrique et/ou de force.

7. Dispositif (10) d'électrostimulation selon la revendication 5 et/ou 6, **caractérisé en ce que** chacune desdites faces opposées des demi-corps (13.3) présente un premier canal respectif et un second canal respectif, dans lequel ledit premier canal s'étend sur toute la longueur du corps (13) assemblé et ledit second canal s'étend latéralement et communique avec ledit premier canal, de sorte que lesdits deux demi-corps (13.3), juxtaposés et fixés l'un à l'autre, fournissent, par l'intermédiaire desdits premier canal et second canal respectifs superposés, ledit premier trou (13.1) et ledit second trou (13.2) du corps de montage (13).

8. Dispositif (10) d'électrostimulation selon l'une quelconque des revendications précédentes, **caractérisé en ce que** ledit support de canule (11.10) présente, sur une surface latérale externe, une disposition linéaire de cavités (12.13), s'étendant dans la direction axiale et configurées comme des encoches d'une échelle graduée, et **en ce que** ledit support de contre-canule (11.20) comprend un doigt élastique (11.221), saillant par rapport à ladite seconde zone d'extrémité (11.22) du support de contre-canule (11.20) et présentant une pointe élastiquement flexible, configurée pour coopérer par encliquetage élastique avec chaque cavité de ladite disposition de cavités (12.13) prévue sur ladite surface latérale dudit support de canule (11.10).

9. Procédé de fabrication d'un dispositif (10) d'électrostimulation à double aiguille percutanée coaxiale selon l'une quelconque des revendications 1 à 7, dans lequel ledit dispositif (10) comprend :
- deux corps tubulaires (11.10, 11.20), en matière plastique, ci-après dénommés respectivement support de canule (11.10) et support de contre-canule (11.20), dans lequel le support de canule (11.10) et le support de contre-canule (11.20) sont raccordés de manière télescopique avec possibilité de coulissement axial relatif ;
- une aiguille à canule (11.1) et une aiguille à contre-canule (11.2), ci-après dénommées respectivement canule (11.1) et contre-canule (11.2), percées axialement, en matériau conducteur électrique, dans lequel la canule (11.1) est supportée fixe et coaxiale par rapport au support de canule (11.10) et la contre-canule (11.2) est supportée fixe et coaxiale par rapport au support de contre-canule (11.20), et dans lequel la contre-canule (11.2) est insérée en disposition coaxiale et est isolée électriquement par rapport à la canule (11.1), avec possibilité de coulissement axial relatif, **caractérisé en ce que** le support de canule (11.10) est inséré au moins partiellement à l'intérieur du support de contre-canule (11.20) de manière mobile en coulissement axial relatif.

10. Procédé selon la revendication 9, **caractérisé en ce qu'**il comprend les étapes suivantes de fabrication dudit support de contre-canule (11.20) avec la contre-canule correspondante (11.2) fixée par rapport audit support de contre-canule (11.20) :
• prévoir une contre-canule (11.2), présentant une première extrémité axiale (11.2a) opposée à une seconde extrémité axiale, dite pointe, non tranchante (11.2') ;
• prévoir un moyen à thermocouple (11.3), configuré pour être inséré et fixé dans ladite contre-canule (11.2) en disposition axiale et présentant une première extrémité axiale (11.31), électriquement conductrice, et une seconde extrémité axiale opposée à ladite première extrémité (11.31) ;
• insérer et fixer ledit moyen à thermocouple (11.3) en disposition axiale dans ladite contre-canule (11.2), en disposant ladite première extrémité axiale (11.31) dudit moyen à thermocouple (11.3) à proximité de ladite première extrémité axiale (11.2a) de la contre-canule (11.2) et ladite seconde extrémité axiale dudit moyen à thermocouple (11.3) à proximité de ladite pointe non tranchante;
• prévoir un premier conducteur électrique isolé (12), ci-après appelé, pour simplifier, premier conducteur (12), présentant une première extrémité électriquement conductrice et une seconde extrémité électriquement conductrice ;
• réaliser un corps de montage (13) de ladite contre-canule (11.2) par rapport audit support de contre-canule (11.20), dans lequel ledit corps de montage (13) est réalisé en matériau électriquement isolant et est pourvu de :
∘ au moins une paroi latérale externe ;
∘ un premier trou (13.1), configuré pour loger une première partie, dite partie terminale, de ladite extrémité axiale (11.2a) de la contre-canule (11.2) ;
∘ un second trou (13.2), traversant et s'étendant entre ledit premier trou (13.1) et ladite au moins une paroi latérale externe dudit corps de montage (13), dans lequel ledit second trou (13.2) est configuré pour loger un premier tronçon d'une première partie dudit premier conducteur (12), dans lequel ledit premier tronçon est contigu à ladite première extrémité électriquement conductrice dudit premier conducteur (12), laquelle est disposée à l'extérieur dudit second trou (13.2) ;
• insérer ladite partie terminale de ladite extrémité axiale (11.2a) de la contre-canule (11.2) dans ledit premier trou (13.1) dudit corps de montage (13), insérer ledit premier tronçon de ladite première partie dudit premier conducteur (12) dans ledit second trou (13.2) dudit corps de montage (13), et juxtaposer ladite première extrémité électriquement conductrice dudit premier conducteur (12) et ladite première extrémité axiale (11.31) dudit moyen à thermocouple (11.3), de façon à réaliser un contact électrique fixe ;
• prévoir un moule configuré pour le moulage par injection de matière plastique fondue et de métal et prévoir dans ledit moule des moyens de formage dudit support de contre-canule (11.20), dans lequel lesdits moyens de formage sont configurés comme cavités d'injection de matière plastique fondue ;
• disposer dans ladite cavité d'injection de matière plastique fondue ledit corps de montage (13) incluant ladite partie terminale de ladite extrémité axiale (11.2a) de ladite contre-canule (11.2), ledit premier tronçon de ladite première partie dudit premier conducteur (12) et ledit contact électrique fixe entre ladite première extrémité électriquement conductrice dudit premier conducteur (12) et ladite première extrémité axiale (11.31) dudit moyen à thermocouple (11.3) ;
• configurer ladite cavité d'injection de matière plastique fondue pour former ledit support de contre-canule (11.20) comme corps tubulaire et pour former, dans ledit support de contre-canule (11.20) :
∘ un diaphragme interne intégral (11.21'), lequel :
▪ comprend un trou axial traversant ;
▪ délimite, dans ledit support de contre-canule (11.20), une première cavité axiale (11.210), dite cavité postérieure, ayant au moins une paroi latérale interne contiguë audit diaphragme (11.21'), au niveau d'une première zone d'extrémité axiale (11.21), et une seconde cavité axiale (11.211), dite cavité antérieure, ouverte vers l'extérieur au niveau d'une seconde zone d'extrémité axiale (11.22), opposée à ladite première zone d'extrémité axiale (11.21), du support de contre-canule (11.20), et
∘ un trou traversant (11.212), lequel :
▪ est réalisé au niveau de ladite extrémité postérieure (11.21), et
▪ présente un axe aligné avec l'axe dudit second trou (13.2) dudit corps de montage (13) et est configuré pour contenir un second tronçon de ladite première partie dudit premier conducteur (12), distal par rapport à ladite première extrémité électriquement conductrice ;
• dériver ledit premier conducteur (12) à l'extérieur de ladite cavité d'injection de matière plastique fondue dudit moule et à l'extérieur dudit moule, au moyen de ladite seconde partie dudit premier conducteur (12) présentant ladite seconde extrémité électriquement conductrice ;
• fermer ledit moule et réaliser l'injection de matière plastique fondue dans ladite cavité d'injection de matière plastique fondue dudit moule, en noyant dans la matière plastique fondue injectée :
∘ ledit corps de montage (13) incluant, au niveau de ladite première cavité axiale (11.210) dudit support de contre-canule (11.20), ladite partie terminale de ladite extrémité axiale (11.2a) de ladite contre-canule (11.2), ledit premier tronçon de ladite première partie dudit premier conducteur (12) et ledit contact électrique fixe entre ladite première extrémité électriquement conductrice dudit premier conducteur (12) et ladite première extrémité axiale (11.31) dudit moyen à thermocouple (11.3) ;
∘ ledit second tronçon de ladite première partie du premier conducteur (12);
∘ une autre partie de ladite extrémité axiale (11.2a) de ladite contre-canule (11.2) contiguë à ladite partie terminale de ladite contre-canule et scellée de manière étanche dans ledit trou axial traversant dudit diaphragme (11.21') dudit support de contre-canule (11.20) ;
• faire durcir la matière plastique fondue injectée et extraire dudit moule ledit support de contre-canule (11.20), dans lequel sont intégrés et fixés ledit corps de montage (13), ladite extrémité axiale (11.2a) de ladite contre-canule (11.2) et ledit second tronçon de ladite première partie du premier conducteur (12).

11. Procédé selon la revendication 10 pour la fabrication du dispositif (10) selon la revendication 5, **caractérisé par** l'étape consistant à former ledit corps de montage (13) en deux demi-corps (13.3) séparés, qui sont ensuite juxtaposés et accouplés par des faces opposées respectives au niveau d'un plan contenant les axes dudit premier trou (13.1) et dudit second trou (13.2) du corps de montage (13).

12. Procédé selon la revendication 10 pour la fabrication du dispositif (10) selon la revendication 6, **caractérisé par** l'étape consistant à prévoir lesdites faces opposées desdits deux demi-corps (13.3) d'évidements respectifs (13.41) et broches correspondantes (13.42) de montage, qui coopèrent pour réaliser l'assemblage du corps de montage (13) par engagement géométrique et/ou de force réciproque.

13. Procédé selon la revendication 10 pour la fabrication du dispositif (10) selon la revendication 7, **caractérisé par** les étapes consistant à prévoir dans chacune desdites faces opposées des demi-corps (13.1) un premier canal respectif et un second canal respectif, dans lequel ledit premier canal s'étend sur toute la longueur du corps (13) assemblé et ledit second canal s'étend latéralement et communique avec ledit premier canal, et à juxtaposer lesdits deux demi-corps (13.3) formant ledit premier trou (13.1) et ledit second trou (13.2) du corps de montage (13) par superposition desdits premier et second canaux respectifs.

14. Procédé selon la revendication 10 pour la fabrication du dispositif (10) selon la revendication 8, **caractérisé par** l'étape consistant à former, sur une surface latérale externe d'une portion d'extrémité (11.12) dudit support de canule (11.1), une disposition linéaire de cavités (12.13), s'étendant dans la direction axiale et configurées comme des encoches d'une échelle graduée, et à former dans ledit support de contre-canule (11.20) un doigt élastique (11.221), saillant par rapport à ladite seconde zone d'extrémité (11.22) du support de contre-canule (11.20) et présentant une pointe élastiquement flexible, configurée pour coopérer par encliquetage élastique avec chaque cavité de ladite disposition de cavités (12.13) formée sur ladite surface latérale externe dudit support de canule (11.10).
